# EUROPEAN PATENT APPLICATION

(11) **EP 2 184 365 A1**
(43) Date of publication of application: **12.05.2010**
(21) Application number: 08305778.6
(22) Date of filing: 05.11.2008
(51) Int. Cl.: C12N 15/90

(54) **Multi-marked diploid yeast strains for use in measuring eukaryotic chromosome instability**

(71) Applicant: Centre National de la Recherche Scientifique- CNRS, 75016 Paris (FR)
(72) Inventor: Dirick, Léon, 34070 MONTPELLIER (FR)
(74) Representative: Blot, Philippe Robert Emile

(57) **Abstract**

The present invention concerns a method for measuring chromosome instability as well as a diploid or polyploid yeast strain suitable for carrying out such a method. The method according to the invention allows detecting chromosome break events with a greatly improved sensitivity. In addition, it also allows detecting chromosome loss events. The method according to the invention is for example useful for evaluating toxicity of pharmaceutical and/or dietary compounds.

## Description

The present invention concerns a method for measuring chromosome instability as well as a yeast strain suitable for carrying out such a method.

Life relies on the faithful transmission of DNA information from generation to generation. It is therefore of importance to assess the mistakes and damages that can occur on chromosomes. For example, cancer progression is often associated with the accumulation of gross chromosomal rearrangements (GCRs), such as translocations, deletion of a chromosome arm, interstitial deletions or inversions. In many instances, GCRs inactivate tumor-suppressor genes or generate novel fusion proteins that initiate carcinogenesis. Due to the importance of GCR during tumorigenesis, both in the initiation and progression steps, the cancer research community has become very interested in the molecular ethiology of GCRs. Yeast in an excellent model organism for this purpose since the cells are normal and contain many human gene homologs, which increase GCR rates when mutated similar to what has been observed in many human cancers. Therefore, yeast has been used a model for studying GCRs, both for better understanding the molecular ethiology of GCRs and for testing the effect of drugs on GCRs during preclinical and clinical trials.

The GCR assay is commonly used to monitor chromosomal instabilities in budding yeast (Chen and Kolodner, 1999, Nat Genet. 23:81-85; Schmidt et al. 2006, Methods Enzymol. 409:462-476; Motegi and Myung, 2007, Methods 41:168-176). In the GCR assay, the loss of two markers (composed of the *CAN1* and *URA3* genes) is selected using both canavanine (Can) and 5-fluorooroticacid (5-FoA). In the haploid wild type strain used for GCR assays, the frequency of GCR events is very low (about 10⁻¹⁰). Thus over 10 billion cells are required for each assay, which imposes strong practical limitations. By introducing the same selectable markers on a different chromosome, Kanellis *et al.* (2007, PLoS Genet. 3:e134) increased the detection limit of the GCR assay by about 250 fold (i.e. a frequency of GCR events of about 9 10⁻⁸). Still, the detection limit is very low. The GCR assay is therefore unsuitable for large numbers of measurements. For instance, the screening of a library of ten thousands compounds using the method of Kanellis *et al.* (2007, PLoS Genet. 3:e134) would require thirty thousand Petri dishes. This makes the use of the GCR assay for high-throughput screenings both expensive and extremely difficult to put in place from a practical point of view. There is therefore a need for an assay for measuring chromosome instability that would exhibit a higher detection limit.

In the GCR assay, the measurement of genetic instability is based on the selection of a gene loss. Several types of DNA damages co-exist, and the main challenge is to overcome the masking effects that one type of event can impose on another. Two parameters are of particular importance: the number of markers and the ploidy of the cell.

When a single gene marker is used, the frequency of point mutations is around 10⁻⁶. For two markers, that frequency drops to 10⁻¹². The existing GCR assay thus uses two markers (the *CAN1* and *URA3* genes). The frequency of chromosome break in the GCR assay is around 10⁻¹⁰. That frequency is thus about 100 fold higher than the frequency of double genes mutations. In other terms, the double genes mutations do not mask chromosome break events.

The GCR assay is done in a haploid context. To be viable, the chromosome break has to occur in a limited distal region of chromosome V, between the *CAN1* marker and the first essential gene (PMC1). This explains why the frequency of chromosome break is so low. However, the use of a diploid or polyploid strain in order to allow functional complementation of essential genes appears not to be an option since it is expected that the chromosome loss events will mask the chromosome break events. Indeed, a diploid or a polyploid cell usually looses an entire chromosome at a frequency that is as high as about 10⁻⁵, i.e., a frequency that is about 100 000 fold higher than the GCR frequency in a haploid cell. Therefore frequency of chromosome loss seems to prohibit to the use of a diploid or a polylploid strain for the measurement of chromosome breaks. Chromosome loss events appearing to be far more frequent than break events, they are thus expected to mask break events in a diploid or a polyploid cell.

### DETAILED DESCRIPTION OF THE INVENTION

A yeast strain that is particularly suitable for measuring chromosome instability (CIN) has been found by the inventors. This strain has the main following features:
- it is diploid or polyploid, i.e. both break and full chromosome loss can occur without loss of viability;
- it comprises two negative selectable markers which allow selecting cells in which a chromosome break or a chromosome loss event has occurred. In the LD 1577 strain, these markers correspond to *URA3* and *CAN1,* which are linked in the distal part of chromosome V. Chromosome instability is measured by lost of these two selectable markers; and
- it comprises a third selectable marker which allows distinguishing between chromosome break and chromosome loss events. In LD 1577, this third marker corresponds to the LEU2 marker, which is located close to centromere V.

It has surprisingly been found that the frequency of break events in such a strain is high, around 10⁻⁵, i.e. similar to chromosome loss frequency. Thus, unexpectedly, chromosome loss events do not mask chromosome break events in such a strain. Furthermore, methods for measuring chromosome instability based on the use of this strain allow distinguishing between chromosome break events (yielding Can 5-FoA resistant LEU+ clones in the case of the LD 1577 strain) and chromosome loss (yielding Can 5-FoA resistant leu- clones in the case of the LD 1577 strain). Thus, in contrast to prior art methods, the method for measuring CIN using such a strain allows detecting chromosome loss events in addition to chromosome break events.

The method in accordance with the invention allows detecting chromosome break events with a greatly improved sensitivity compared to the prior art assays. It is 20 000 fold more sensitive than the original GCR assay disclosed by Chen and Kolodner (1999, Nat Genet. 23:81-85), and 100 fold more sensitive than the GCR assay disclosed by Kanellis et al. (2007, PLoS Genet. 3:e134).

Due to its increased sensitivity, the method in accordance with the invention can be performed starting from a single colony. It can therefore be carried out as a clonal assay, which can thus detect early events in chromosome instability rather than amplified non-independent events.

Further due to its increased sensitivity, the method according to the invention can be scaled down. Multiple samples can be run easily, which allows repetition and accurate statistical analysis of the data. In addition, the method according to the invention may be performed using multi-well plates rather than Petri dishes. It thus allows robotic handling and drastically reduces the cost.

The method in accordance with the invention being applicable to very small samples, it is suitable for screening purposes. It may for example be used to assess geno-toxicity and/or geno-protection of large numbers of products or mutants. For example, the screening of a library of ten thousands compounds using the method according to the invention would only require about three hundred 96-well plates, which is a great improvement compared to the thirty thousand Petri dishes that would be required using the method disclosed by Kanellis et al. (2007, PLoS Genet. 3:e134).

The strain according to the invention is thus a very useful tool to analyze chromosomal instabilities in all kinds of physical, chemical or genetic conditions. More specifically, the method according to the invention is for example useful for evaluating toxicity of pharmaceutical and/or dietary compounds.

### Yeast strain according to the invention

The invention is directed to a yeast strain comprising a first and a second selectable marker located on a chromosome, wherein said first and second selectable markers are negative selectable markers, **characterized in that**:
- said yeast strain is diploid or polyploid;
- said yeast strain further comprises a third selectable marker located on said chromosome; and
- said first, second and third selectable markers are only present on one of the homologous copies of said chromosome.

As used herein, the term "selectable marker" refers to an allele, the presence or the absence of which can be selected. More specifically, the presence or the absence of said marker can be selected in a cell either by addition of a selective compound to the medium on which the cell is grown, or by removal of a selective compound from the medium on which the cell is grown. Selectable markers are well-known in the art and include, e.g. *CAN1, URA3* and *LEU2*. The selectable marker according to the invention may for example correspond to an auxotrophic marker (e.g. *LEU2,* which confers the capacity to a cell to grow in the absence of leucine) or to an allele conferring resistance or sensitivity to a drug (e.g. *CAN1* and *URA3,* which confer sensitivity to canavanine and to 5-fluorooroticacid, respectively). Selectable markers are also commonly referred to as "selection markers" in the art.

As used herein, a "positive selectable marker" refers to a selective marker of which the presence can be selected. A "negative selectable marker" refers to a selectable marker of which the absence can be selected (i.e. the presence of the marker can be counter-selected).

Examples of positive selectable markers include e.g. *LEU2,* the presence of which can be selected on a medium lacking leucine, *HIS3,* the presence of which can be selected on a medium lacking histidine, *TRP1*, the presence of which can be selected on a medium lacking tryptophan, *ADE2*, the presence of which can be selected on a medium lacking adenine, and *kanMX4*, the presence of which can be selected on a medium comprising G418.

Examples of negative selectable markers include *CAN1* and *URA3,* the absence of which can be selected on media comprising canavanine and 5-fluorooroticacid, respectively.

As used herein, a gene indicated in capital letters refers to a wild-type, functional allele. A gene indicated in small letters refers to a mutated, non functional allele.

The first and second selectable markers correspond to negative selectable markers, i.e. markers that can be counter-selected in the presence of a selective compound. These first and second markers may for example correspond to *CAN1* and *URA3,* which can be counter-selected in the presence of canavanine and 5-fluorooroticacid, respectively. The third selectable marker may either correspond to a positive selectable marker or to a negative selectable marker. The third selectable marker preferably corresponds to a positive selectable marker such as e.g. *LEU2.* Of course, the first, second and third selectable markers necessarily correspond to different markers, and they are necessarily absent from all other chromosomes, otherwise it would be impossible to select their presence or their absence on the chromosome of interest.

The first, second and third selectable markers are only present on one of the homologous copies of said chromosome. For example, when the strain is diploid, the first, second and third selectable markers are only present on one of the two homologous chromosomes. In other words, all three markers are located not only on the same chromosome, but also on the same copy of the chromosome.

The first and second selectable markers are located on the same arm of the chromosome (i.e., on the same side of the centromere).

The yeast strain according to the invention is suitable for measuring chromosome instability (CIN), i.e., the first and second selectable markers are located on the chromosome in such a way as to allow selecting cells in which a chromosome break or a chromosome loss event has occurred, and the third selectable marker is located on the chromosome in such a way as to allow distinguishing between chromosome break and chromosome loss events.

The distance between the centromere and the first or second selectable marker that is located closest to the centromere defines the scorable breakage zone. The first and second selectable markers may for example be located at a distance of at least 50, 100, 200, 300, 400, 500, 800 or 1000 kb to the centromere. Preferably, said distance is of at least 100 kb. This distance can be increased or decreased by the skilled in the art, depending on the chromosome on which the markers are located and on the chromosome break frequency that is wished for.

In a preferred embodiment, the first and second selectable markers are located in the distal part of the chromosome, i.e. in the most centromere-distal moiety of a chromosome arm. More preferably, they are located in the most centromere-distal third of the chromosome arm. Most preferably, they are located in the most centromere-distal quarter of the chromosome arm.

Also preferably, the first and second selectable markers are located at a distance of at least (or of about) 5, 10, 20, 50 or 100 kb to the telomere in order to avoid a possible gene repression effect.

Also preferably, the first and second selectable markers are linked together, i.e. they exhibit a recombination frequency of less than 50%. More preferably, they exhibit a recombination frequency of less than 40%, 30%, 20%, 10%, 5% or 1%. A short distance between the first and second selectable markers allows the amplification of the first and second selectable markers as a cassette by PCR, and their transfer to a different chromosome location if desired. In addition, putting them further apart would reduce the scorable breakage zone. In a preferred embodiment, the distance between the first and second selectable marker may be of less than 50, 40, 30, 25, 20, 15, 10 or 5 kb.

The third selectable marker is located on the chromosome in such a way as to allow distinguishing between chromosome break and chromosome loss events. More specifically, the third selectable marker is located close to the centromere, in such a way as to be lost upon chromosome loss events but not (or rarely) upon chromosome break events.

In a preferred embodiment, the third selectable marker is located in the most centromere-proximal moiety of a chromosome arm. More preferably, it is located in the most centromere-proximal third of the chromosome arm. Most preferably, it is located in the most centromere-proximal quarter of the chromosome arm. The third selectable marker may for example be located at a distance of less than 50, 40, 30, 20, 10 or 5 kb to the centromere. Preferably, it is located at a distance of less than 30 kb to the centromere.

The third selectable marker may or may not be located on the same arm of the chromosome than the first and second selectable markers. In a preferred embodiment, the third selectable marker is located on the opposite arm of the chromosome as compared to the first and second selectable markers. This prevents the risk of a chromosome break event occurring between the third selectable marker and the centromere being counted as a chromosome loss event. Alternatively, the third selectable marker is located on the same chromosome arm as the first and second selectable markers. In this case, the distance between the third selectable marker and the first and second selectable markers is preferably of at least 20, 40, 50, 60, 80 or 100 kb.

Thus a preferred yeast strain in accordance with the invention is a strain in which:
- the first and second selectable are located at a distance of at least 50, 100, 200, 300, 400, 500, 800 or 1000 kb to the centromere;
- the first and second selectable are located at a distance of at least 5, 10, 20 or 50 kb to the telomere;
- the third selectable is located at a distance of less than 50, 40, 30, 20, 10 or 5 kb to the centromere;
- the third selectable is located on the opposite arm of the chromosome relative to the first and selectable marker, or, when located on the same arm, at a distance of at least 10, 25, 50, 60, 70, 80 or 100 kb to the first and second selectable markers; and/or
- the distance between the first and second selectable marker is of less than 50, 25, 10 or 5 kb.

The yeast strain in accordance with the invention is preferably diploid, i.e. it comprises two homologous copies of each of the 32 chromosomes. The diploid strain is preferably a MATa/α strain, i.e., it does not result from an illegitimate mating phenomenon. Alternatively, the strain may correspond to a polyploid strain instead of a diploid strain. In such a case, the three selectable markers are only present on one of all homologous copies of the chromosome.

The selectable markers may be located on any of the chromosomes. They may for example be located on chromosome V or on chromosome XV. The skilled in the art may choose to study a given chromosome, breakage zones of particular interest (for example, slow replicating zones or zones known to be fragile) or add an heterologous sequence coming from a different organism to test the fragility of particular sites (see e.g. page 174 of Motegi and Myung, 2007, Methods, 41:168-176).

The yeast strain in accordance with the invention may further comprise one or more deleted gene, disrupted gene, mutated gene, exogenous gene and/or gene placed under the control of an exogenous promoter allowing its overexpression. Such a yeast strain may be used to test whether said gene increases or decreases chromosome instability.

The yeast strain in accordance with the invention may also comprise additional selectable markers (e.g. markers conferring resistance to an antibiotic). The additional markers may or may not be located on the same chromosome as the selectable markers in accordance with the invention.

In a preferred embodiment, the yeast strain in accordance with the invention is not capable of entering meiosis. Such a strain may for example be obtained by deleted a gene essential for meiosis such as e.g. *spo11* or a *ime2*. In a preferred embodiment, the strain is deleted for *spo11.* Indeed, *spo11* is meiotic-specific (i.e. the deletion does not affect mitosis). *spo11* mutants undergo sporulation, but all the spores are not viable.

Preferred strains according to the invention are described in further details in Example 1. A most preferred strain in accordance with the invention is the strain referred to as LD 1577 as well as strains derived therefrom. These strains are diploid strains carrying the *CAN1* and *URA3* negative selectable markers, about 8 to 8.5 kb apart, on the left arm of chromosome V. Thus one copy of the chromosome V comprises:
- the wild-type *CAN1* allele, which is located at its natural locus;
- the wild-type *URA3* allele, which integrated at the *hxt13* locus; and
- the wild-type *LEU2* allele, which integrated at the *ura3* locus.

The other chromosome V of such strains comprises a mutated *can1* allele, a mutated *ura3* allele and a wild-type *HXT13* allele.

In such strains:
- the first, second and third selectable markers are located on the same arm of chromosome V (the left arm);
- the first and second selectable are located at a distance of about 110 kb to the centromere (*CEN5*), and of about 22 kb to the left telomere of chromosome V; and
- the third selectable is located at a distance of about 30 kb to *CEN5*, and of about 83 kb to the first and second selectable markers.

The invention is further directed to a yeast chromosome comprising a first and a second negative selectable marker located on the same arm of said chromosome **characterized in that** said chromosome further comprises a third selectable marker. These selectable markers are located on the chromosome as described hereabove for the yeast strain in accordance with the invention. More specifically, the first and second selectable markers are located in the distal part of the chromosome, preferably linked together, and the third selectable marker is located close to the centromere.

### Methods for measuring chromosome instability according to the invention

The yeast strain in accordance with the invention can advantageously be used for measuring chromosome instability. Thus the invention is directed to a method for measuring chromosome instability comprising the steps of:
a) providing a yeast strain according to the invention;
b) plating cells of said yeast strain on a medium selecting cells in which said first and second selectable markers have been lost; and
c) replicating said cells on a medium selecting cells in which said third selectable marker is either present or absent.

In this method, the detection of a colony growing on the medium of step (b) indicates that a chromosome loss or a chromosome break event has occurred in the cells of said colony.

As used herein, the term "chromosome instability" (CIN) refers both to chromosomal break and to chromosome loss. As further detailed below, the method may also be used to measure either chromosome break or chromosome loss.

As used herein, the term "non-selective medium" refers to a medium on which a given yeast strain can grow irrespective of the absence or the presence of the first, second and third selective markers on its chromosomes. For example, a medium comprising leucine, and devoid of canavanine (Can) and of 5-fluorooroticacid (5-FoA), is a non-selective medium for an experiment carried out starting from a strain comprising the *URA3, CAN1* and *LEU2* selectable markers.

The medium of step (b) selects cells in which said first and second selectable markers have been lost. On the other hand, the medium of step (b) is a non selective medium in respect of the third selectable marker (i.e. the cells must be able to grow irrespective of the presence or the absence of the third selectable marker). For example, a medium comprising leucine, canavanine (Can) and 5-fluorooroticacid (5-FoA) can be used at step (b) when the experiment is carried out starting from a strain comprising *URA3* and *CAN1* as the first and second selectable markers and *LEU2* as the third selectable marker.

The medium of step (c) selects cells in which the third selectable marker is present or absent. More specifically, when the third selectable marker is a positive selectable marker, cells are selected for the presence of said marker. On the contrary, when the third selectable marker is a negative selectable marker, cells are selected for the absence of said marker. For example, a medium devoid of leucine can be used at step (c) when the experiment is carried out starting from a strain comprising *LEU2* as the third selectable marker.

After provision of a yeast strain according to the invention, step (a) preferably further comprises the step of growing the yeast strain on a non-selective medium. The yeast strain may either be grown in a liquid medium or on a solid medium. During this growth, chromosome instability events may occur. The non-selective medium may for example consist of a rich YPD medium comprising 2% glucose. The cells are preferably grown at about 20-42°C, 20-37°C, 25-35°C or 30°C.

At step (a), the yeast strain may be plated on any type of support such as e.g. Petri dishes or multi-well plates. The medium may be liquid or solid. Petri dishes are particularly suitable for performing clonal assays. On the other hand, multi-well plates are particularly suitable for performing assays with a large number of samples, for example in the frame of a screening.

When a clonal assay is carried out (i.e., an assay performed with cells derived from a single colony), the strain is preferably grown on a Petri dish until isolated colonies are obtained. Then, a representative colony can be picked. Thus, after growth of the strain on a non-selective medium, step (a) may further comprise the steps of isolating a colony and suspending the cells of said colony in a solution. Said solution may for example consist of water.

When multi-well plates are used, the wells may for example comprise 200 µl liquid medium and be inoculated with about 200 cells/well. After growth to saturation, strains usually reach around 100 million cells/ml. 5 µl of that culture (i.e. about 5.10⁵ cells) may then be used for plating at step (b). Those numbers can easily be adapted by the skilled in the art.

Upon provision of the strain in accordance of the invention and before cultivation on the non-selective medium, the strain may first be cultivated on a selective medium into ensure that all selectable markers are present. For example, when the experiment is carried out with the LD 1577 strain, the strain may be cultivated on a medium lacking uracil and leucine.

The method according to the invention may be used to determine whether a candidate compound increases or decreases chromosome instability. In the frame of this embodiment, the non-selective medium of step (a) further comprises the candidate compound to be tested. The chromosome instability of the strain in the presence of said candidate compound may then be compared to the chromosome instability of the same strain in the absence of said candidate compound.

The method according to the invention may also be used to determine whether a candidate gene increases or decreases chromosome instability. In the frame of this embodiment, the yeast strain provided at step (a) is a strain wherein the candidate gene is deleted, disrupted, inactivated or overexpressed. The chromosome instability of the strain in which said candidate gene is deleted, disrupted, inactivated or overexpressed may then be compared to the chromosome instability of the same strain in which said candidate gene is in its wild-type form. The overexpressed candidate gene may correspond to a heterologous gene that is absent from the wild-type yeast genome.

As used herein, the terms "candidate gene", "candidate compound" and "candidate physical parameter" refer to genes, compounds and physical parameters that are susceptible of playing a role in chromosome instability. The strains and methods may be used to determine whether a candidate gene, a candidate compound or a candidate physical parameter increases or decreases chromosome instability. For example, the skilled in the art may determine whether a candidate gene plays a role in chromosome instability by comparing the chromosome instability measured in a yeast strain according to the invention in which said candidate gene is deleted or over-expressed with the chromosome instability measured in a yeast strain according to the invention that comprises a wild type candidate gene. Similarly, the skilled in the art may determine whether a candidate compound plays a role in chromosome instability by comparing the chromosome instability measured in yeast strains according to the invention that are grown in the presence and in the absence of the candidate compound, respectively.

The candidate compound may correspond to any compound such as e.g. a chemical molecule (for example a small molecule), a protein, a peptide, a lipid, a DNA molecule, a RNA molecule, an antisense polynucleotide, a small interfering RNA, an aptamer or an antibiotic. The candidate compounds may for example be manufactured and/or used by industries in the field of food, agribusiness, agro-chemistry and/or pharmaceuticals.

In a preferred embodiment, the candidate compound corresponds to a compound intended for a pharmaceutical use such as, e.g., a compound undergoing preclinical or clinical trials for the treatment of a disease.

In another preferred embodiment, the candidate compound corresponds to a dietary compound, a nutrient and/or a food component. Indeed, the methods in accordance with the invention are useful for evaluating the effect of a dietary compound on genetic instability and thus evaluating their potential genotoxic effect.

In still another preferred embodiment, the candidate compound corresponds to a candidate chemical compound (e.g. manufactured and/or used in the field of agro-chemistry), and the method according to the invention is carried out to test the potential toxicity of such candidate chemical compounds.

The candidate gene may correspond to any gene. It may for example correspond to a gene of which the human homolog plays a role in a disease such as e.g. cancer.

The candidate physical parameter may for example correspond to light or radiation. Alternatively, the candidate physical parameter may correspond to a physiological or a growth parameter such as temperature, nutrient composition growth conditions, etc.

Step (b) allows selecting cells wherein a chromosome instability event has occurred. At step (b), the cells are plated on a solid medium in order to count the number of colonies obtained. Thus plating on a Petri dish is preferred. When multi-well plates are used at step (a), rectangular Petri dishes with the same size as the multi-well plates may be used, thus allowing automatic transfer from the medium of step (a) to the medium of step (b). A chosen number of cells may be plated on a Petri dish (e.g. about 0.1-10 or 1-5 10⁵ cells) and allowed to grow for e.g. 1-10, 1-5 or 1-3 days. The cells are preferably grown at about 20-42 °C, 20-37 °C, 25-35 °C or 30°C. The number of colonies growing on the Petri dishes is indicative of the number of chromosome instability events that have occurred among the number of plated cells.

Step (c) allows distinguishing between chromosome loss and chromosome break events. This is determined by replicating the cells growing at step (b) on a medium allowing determining whether the third selectable marker is still present or whether it is lost as well.

The third selectable marker preferably corresponds to a positive selectable marker. In this case, the medium of step (c) selects cells in which the third selectable marker is present. In the frame of this embodiment:
- the detection of a colony growing both on the medium of step (b) and on the medium of step (c) indicates that a chromosome break event has occurred in the cells of said colony; and/or
- the detection of a colony growing on the medium of step (b) but not on the medium of step (c) indicates that a chromosome loss event has occurred in the cells of said colony.

Alternatively, the third selectable marker may correspond to a negative selectable marker. In this case, the medium of step (c) selects cells in which the third selectable marker is absent. In the frame of this embodiment
- the detection of a colony growing both on the medium of step (b) and on the medium of step (c) indicates that a chromosome loss event has occurred in the cells of said colony; and/or
- the detection of a colony growing on the medium of step (b) but not on the medium of step (c) indicates that a chromosome break event has occurred in the cells of said colony.

Once the number of chromosome break and the number of chromosome loss events have been measured, the frequency and/or the rate of chromosome instability (and/or of each one of these events) can easily be calculated by the skilled in the art. For example, the number of CIN events per 10⁶ cells (referred to as "the CIN frequency") may be calculated. Alternatively, the rate of chromosome instability may be measured using the method that is disclosed in the paragraph entitled "2.4. Real calculation" at page 170 of Motegi and Myung (2007 Methods 41:168-176). This method, referred to as the "Lea-Coulson median estimator", as well as other calculation methods that may be used, is reviewed in Foster (2006, Methods Enzymol. 409:195-213).

In order to make sure that the loss of all three markers is due to a chromosome loss event and not to a meiosis event, it is possible to test whether the colonies detected at step (b) and/or (c) are still diploid. Thus, the method according to the invention may further comprise the step of detecting haploid cells growing at step (b) and/or (c) by a mating type-test. However, this step is optional since it has been shown that the number of meiosis event is negligible compared to the number of chromosome loss events (see Example 2).

The invention is further directed to the use of a strain according to the invention or to a method according to the invention for determining whether a candidate gene, a candidate compound and/or a candidate physical parameter increases or decreases chromosome instability (see e.g. Example 3).

A preferred embodiment of the invention is directed to a method of determining whether a candidate compound or a candidate physical parameter increases or decreases chromosome instability comprising the steps of:
i. measuring chromosome instability in the presence of said candidate compound or candidate physical parameter;
ii. measuring chromosome instability in the absence of said candidate compound or candidate physical parameter;
iii. comparing the chromosome instability measured at step (i) and (ii);
wherein:
- chromosome instability is measured according to any one of the methods described herein;
- the measure of a higher chromosome instability at step (i) than at step (ii) indicates that said candidate compound or candidate physical parameter increases chromosome instability; and
- the measure of a lower chromosome instability at step (i) than at step (ii) indicates that said candidate compound or candidate physical parameter decreases chromosome instability.

This method may also be used to determine whether a candidate compound or a candidate physical parameter increases or decreases chromosome break or chromosome loss. The candidate compound is preferably added to the non-selective medium used at step (a) of the method for measuring chromosome instability according to the invention. The candidate physical parameter is preferably applied during the growth in the non-selective medium of step (a) of the method for measuring chromosome instability according to the invention.

Another preferred embodiment of the invention is directed to a method of determining whether a candidate gene increases or decreases chromosome instability comprising the steps of:
i. measuring chromosome instability in a strain comprising said candidate gene;
ii. measuring chromosome instability in a strain wherein said candidate gene is deleted or disrupted;
iii. comparing the chromosome instability measured at step (i) and (ii);
wherein:
- chromosome instability is measured according to any of the methods described herein;
- the measure of a higher chromosome instability at step (i) than at step (ii) indicates that said candidate gene increases chromosome instability; and
- the measure of a lower chromosome instability at step (i) than at step (ii) indicates that said candidate gene decreases chromosome instability.

This method may also be used to determine whether a candidate gene increases or decreases chromosome break or chromosome loss. The two strains are preferably identical except for the presence or the absence of said candidate gene. Alternatively, the method may be performed with a strain overexpressing said candidate gene at step (i), and a strain comprising a wild-type candidate gene at step (ii).

All references cited herein, including journal articles or abstracts, published or unpublished patent application, issued patents or any other references, are entirely incorporated by reference herein, including all data, tables, figures and text presented in the cited references.

The invention will be further evaluated in view of the following examples and figures.

### BRIEF DESCRIPTION OF THE FIGURES

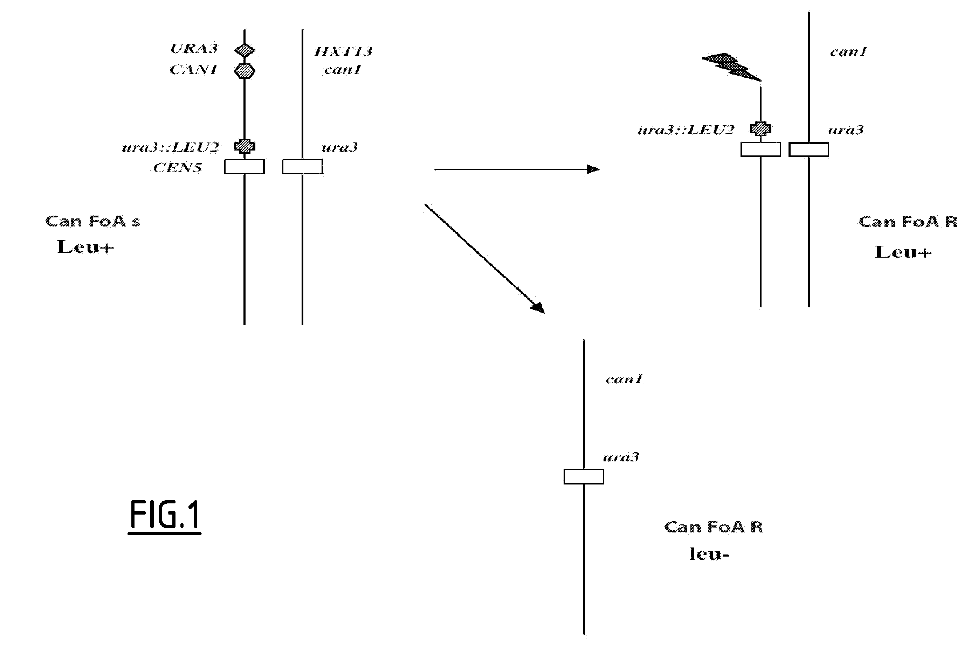

Figure 1 corresponds to a schematic view of a strain in accordance with the invention. Genome instability is measured by positive selection of marker loss *(URA3* and *CAN1* genes), on the distal arm of chromosome V. Combined loss of *URA3* and *CAN1* genes leads to colonies resistant to the drugs 5-FoA and canavanine (Can FoA resistant). In a CINA strain, this phenotype can either be due to chromosome break (Leu+) or to full chromosome loss (leu-).

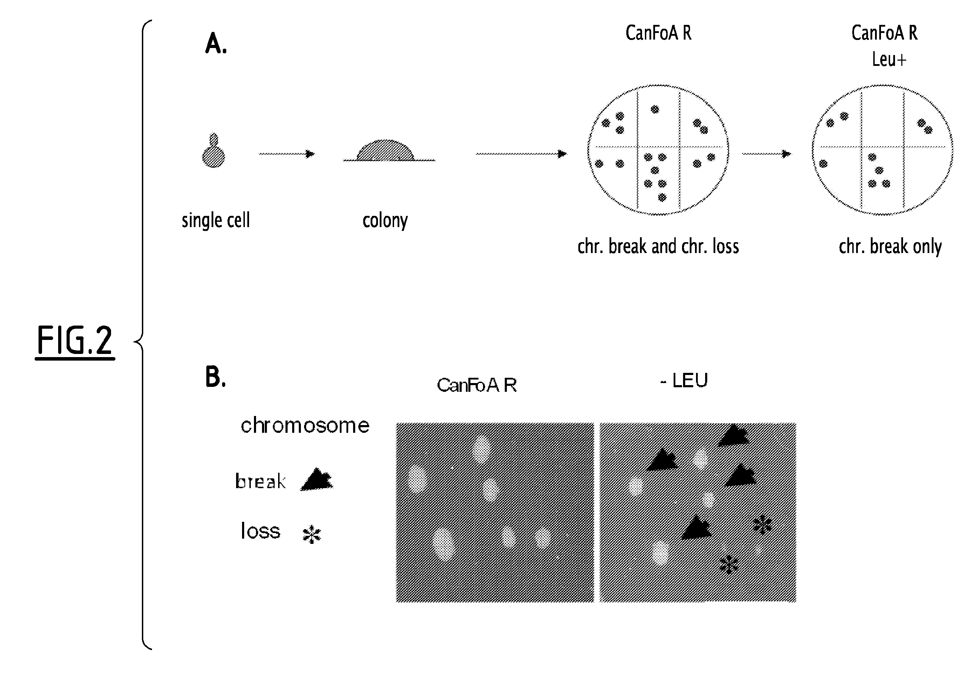

Figure 2 is a scheme of an assay in accordance with the invention (CIN assay). **A.** Starting from single cells, clones are grown and chromosome instability events detected on CanFoA plates. Replica on plates lacking leucine allows to distinguish between chromosome break (CanFoA resistant, LEU+) and chromosome loss (Can FoA resistant, leu-). **B.** Picture of colonies growing on CanFoA plates and on plates lacking leucine.

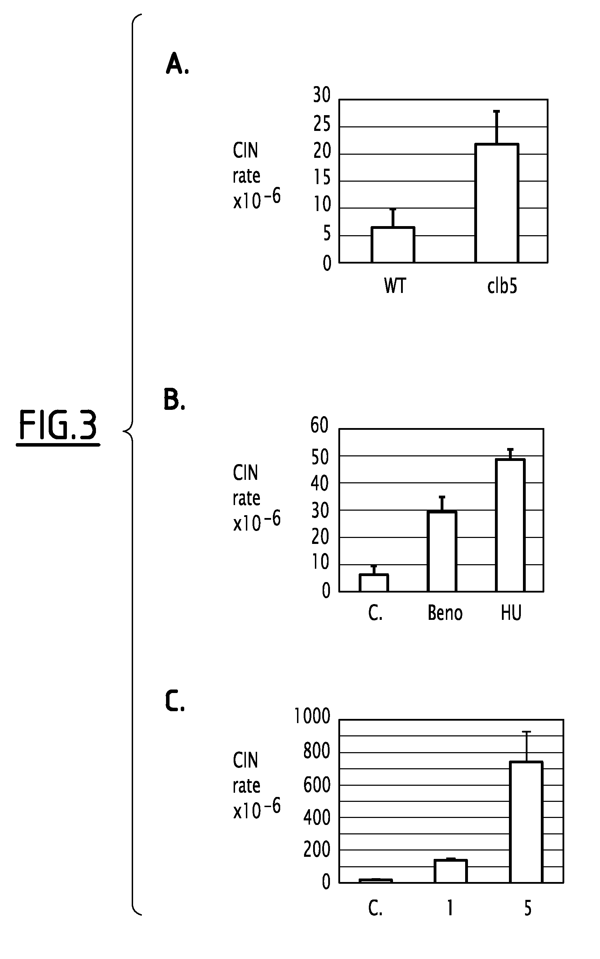

Figure 3 shows the CIN rates of various strains in accordance of the invention, in the presence of various compounds. The strains grown to colonies on YEPD at 30°C. Each assay was repeated 18 times. **A.** CIN rate in the wild-type CINA strain (LD1577; WT) and in a strain comprising an additional mutation of the S-phase cyclin *clb5* (*clb5*). **B**. CIN rate in the wild-type CINA strain (LD1577). The strain was grown on YEPD plates (C), on YEPD plates in the presence of benomyl (12 µg/l) (+Beno), or on YEPD plates in the presence of hydroxyurea (50 mM) (+HU). C. CIN rate in the wild-type CINA strain (LD1577). The strain was grown on YEPD plates (C), or on YEPD plates in the presence of increasing concentrations of Zeocin (1 or 5 µg/ml).

### EXAMPLES

### EXAMPLE 1: Construction of strains in accordance with the invention

### 1.1. The wild type CINA strain

*A LEU2* wild type gene was integrated at the mutated *ura3* locus, close to *CEN5*, in the RDK 3615 Mata strain carrying a *URA3-CAN1* selection cassette (Myung et al., 2001, Nature, 411:1073-6).

Proper integration of the marker was verified by southern blot and genetic analysis. The resulting strain is referred to as LD 1520 and has the following genotype: *MATa, ura3-52::LEU2, leu2Δ1, trp1Δ63, his3Δ200, lys2ΔBgl, hom3-10, ade2Δ1, ade8, hxt13::URA3 CAN1* (S288c).

The mating partner carrying a *can1* mutant allele marked with kanMX4 marker is referred to as LD 1571 and has the following genotype: *MATalpha his3 ::1 leu2::0 met15::0 ura3::0 can1::kanMX 4* (Euroscarf).

LD 1520 was mated with LD 1571 and a diploid zygote was pulled of. This led to the strain referred to as LD 1577, i.e. the wild type CINA strain (for a schematic view of this strain, see Figure 1).

### 1.2. Mutant CINA strains

To generate mutant strains for CIN assays, a mutation in a given gene was introduced separately in haploid parents by genetic crosses.

One parent was LD 1847, a *Matalpha* version of LD 1520 (*ura3-52::LEU2 hxt13::URA3 CAN1*).

The chosen mutation was separately introduced in the LD 1607 Mata strain carrying a natural untagged *can1* mutation. Strain LD 1607 has the genotype: *Matalpha leu2::0 lys2::0 met15::0 trp1::63 ura3::0 can1.* Crossing of such parents leads to homozygous mutant diploid strain with the appropriate heterozygous selection markers for chromosome instability assay.

To generate ρ*O* strains, cells were grown to saturation in liquid YPD medium + ethidium bromide (10mg/ml) and plated on YPD plates for individual colonies. Clones were checked for growth defects on a non-fermentable carbon source (YPG 2% glycerol) and absence of mtDNA was confirmed by DAPI staining.

For natural *petite*, untreated cells were plated on YPD 2% glucose in the absence of treatment or selection and small growing colonies were screened as above (YPG-).

Strains with a defined nuclear-encoded mitochondrial defect (*mip1, mrps28*) were checked likewise.

The strains were grown at 30°C on YPD medium (2% glucose) unless indicated otherwise.

### 1.3. Genotype of strains used in Examples 2 and 3

| **strain** | **genotype** | **Comment** |
|---|---|---|
| 1459 | *MATa, ura3-52, leu2Δ1, trp1Δ63, his3Δ200, lys2ΔBgl, hom3-10, ade2Δ1, ade8, hxt13::URA3 CAN1* | |
| 1472 | *MATa, ura3-52, leu2Δ1, trp1Δ63, his3Δ200, lys2ΔBgl, hom3-10, ade2Δ1, ade8, hxt13::URA3 ρO* | |
| 1496 | *MATa, ade2-1, ade3, trp1-1, can1-100, leu2-3112, his3-11, 15, ura3, GAL, psi+ mrps28::HIS3 RAD52:GFP* ρ*0* | |
| 1520 | *MATa, ura3-52::LEU2, leu2Δ1, trp1Δ63, his3Δ200, lys2ΔBgl, hom3-10, ade2Δ1, ade8, hxt13::URA3 CAN1* | |
| 1571 | *MATα, his3 ::1 leu2::0 met 15::0 ura3::0 can1::kanMX 4 TRP+* | Obtained from Euroscarf |
| 1577 | ^{∼}/*MATaα, ura3-52::LEU2, leu2Δ1, trp1Δ63, his3Δ200, lys2ΔBgl, hom3-10, ade2Δ1, ade8, hxt13::URA3, CAN1* / *his3 ::1 leu2::0 met15::0 ura3::0 can1::kanMX 4* | Referred to as CINA WT |
| 1607 | *MATα*/*eu2::0 lys2::0 met15::0 trp1::63 ura3::0 can 1* | Natu ral *can1* mutant |
| 1690 | *MATa*/*α, ura3-52::LEU2, leu2Δ1, trp1Δ63, his3Δ200, lys2ΔBgl, hom3-10, ade2Δ1, ade8, hxt13::URA3, CAN1* / *his3 ::1 leu2::0 met15::0 ura3::0 can1::kanMX 4* ρ*0* | CINA ρ*0* |
| 1719 | *MA Ta RAD52:GFP (unmarked) ADE+* | |
| 1721 | *MATα, RAD52:GFP (unmarked) ADE+ ρ0* | |
| 1779 | *MATa*/*α, his3 ::1 leu2::0 met15::0 ura3::0 trp1 mip1::kanMX4 hxt13::URA3 CAN1* / *his3 ::1 leu2::0 met15::0 ura3::0 can 1::kanMX 4 mip1::kanMX4 (*ρ*0)* | CINA *mip1 (*ρ*0)* |
| 1781 | *MATa*/*α, ura3-52::LEU2, leu2Δ1, trp1Δ63, his3Δ200, lys2ΔBgl, hom3-10, ade2Δ1, ade8, hxt13::URA3, CAN1* / *his3 ::1 leu2::0 met15::0 ura3::0 can1::kanMX 4 pet (*ρ*0)* | CINA Natural *petite* of 1577 |
| 1822 | *MATa*/*α, his3 ::1 leu2::0 met15::0 ura3::0 tsa1::kanMX 4 ura3-52::LEU2 hxt13::URA3 CAN1* / *his3::1 leu2::0 met15::0 ura3::0 tsa1::kanMX 4 can 1* | CINA *tsa1* |
| 1829 | *MATa*/*α, his3 ::1 leu2::0 met15::0 ura3::0 tsa1::kanMX 4 ura3-52::LEU2 hxt13::URA3 CAN1* / *his3 ::1 leu2::0 met15::0 ura3::0 tsa1::kanMX 4 can 1* ρ*0* | CINA *tsa1 ρ0* |
| 1847 | *MATα, ura3-52::LEU2 hxt13::URA3 CAN1 his3 TRP+* | |

### EXAMPLE 2: The CIN assay in accordance with the invention

### 2.1. Protocol

Dilutions of the CINA strain were done on the plate of choice (unless stated, rich YPD 2% glucose plates). They were grown at 30°C to give rise to isolated colonies (around one hundred colonies/plate). When grown to visible colonies (10⁶ cells for small colonies, up to around 10⁷ cells for large ones), a representative colony was picked and measured both for cell number and for cell size using a Casy Counter. Individual colonies were picked and suspended in water (20 µl). The chosen number of cells (around 1-5 10⁵ cells) was plated and allowed to grow for 2-3 days at 30°C on selective plates (Can 5-FoA) in order to select for chromosome instability events. Plating adjustment of cell number were made depending on the genomic instability of individual strains.

Using this protocol, six assays could easily be done on a single small Petri dish. Chromosome break could be distinguished from full chromosome loss by replica plating of Can 5-FoA R clones on plates lacking leucine (see Figure 2). Can 5-FoA resistant Leu+ clones result from chromosomal break between the *URA3-CAN1* and *LEU2* markers (see Figure 1) while Can 5-FOA resistant leuclones result from full chromosome loss of the homolog carrying the selective markers (see Figure 1).

It may be noted that low frequency meiosis could lead to false positives (Can 5-FoA resistant). Unlike the genuine diploid clones, those are haploid and can be detected using mating type-tests. Such maters can stem either from meiotic recombination or from the loss of chromosome III, the chromosome carrying the mating type locus. The occurrence of meiosis was thus assessed for a WT CINA strain. Positive maters ranged from 1 to 5% of total Can 5-FoA resistant clones. Thus meiosis is negligible compared to the global percentage of chromosome instability values. In addition, RD cells do not sporulate and hence do not suffer from this small bias.

The rate of chromosome instability was calculated using the Lea-Coulson median estimator (see e.g. Foster, 2006, Methods Enzymol. 409:195-213).

### 2.2. Results

When the wild type CINA strain is grown on rich YEPD medium, the strain displayed a chromosome instability of about 6 marker losses per million of cells (6 10⁻⁶ Can 5-FOA resistant, see Figure 3A). About half of the losses were due to chromosome break (Can 5-FOA resistant, 60% LEU+) and half to chromosome loss (Can 5-FOA resistant, 40% leu-).

As a control, drugs and mutations known to affect chromosome stability in yeast were used (see Figure 3A and 3B).

Benomyl (12 µg/ml) increased CIN by 5 fold (29 +/- 5 10⁻⁶). Most events (Can 5-FoA^{R}) were due to total chromosome loss (10% LEU+, 90% leu-). This was expected for a microtubule poisoning agent.

Growth in presence of zeocin, which creates double strand breaks, strongly increased CIN in a dose-dependent manner : 20 fold (130 +/- 11 10⁻⁶) at 1µg/ml of zeocin, and 120 fold (740 +/- 185 10⁻⁶) at 5 µg/ml of zeocin. As expected, there was a strong bias towards chromosome breaks (over 90% LEU+).

Hydroxyurea, an inhibitor of ribonucleotide reductase Rnr1, slows down replication forks (Alvino et al. 2007 Mol Cell Biol. 27, 6396-6406) and increased chromosome instability 8 fold.

CIN was increased 3-4 fold (22 +/- 6 10⁻⁶) in a cell cycle mutants *clb5*. The CIN assay thus also allows detecting genomic destabilization.

Hence, the CIN assay is a sensitive assay that allows the detection of chromosome instability. In addition, it is a clonal assay that can be done on small samples.

### 2.3. Discussion

CINA is a highly sensitive yeast assay to quantify chromosome instability.

Until now, the GCR (gross chromosomal rearrangement) assay was used to monitor chromosomal instabilities in budding yeast. The loss of two markers (composed of the *CAN1* and *URA3* genes) was positively selected using both canavanine and 5-FoA drugs. In a haploid wild type strain, the frequency of such events is very low, around 10⁻¹⁰ (for a recent review, see Motegi and Myung, 2007, Methods 41, 168-176).

The CIN assay presents greatly improved sensitivity and supplemental features compared to GCR. The CIN assay is 20 000 fold more sensitive (6 10⁻⁶ for a wild type) than the original GCR assay, and 100 fold more sensitive compared to an improved GCR version (Kanellis *et al.* 2007 PLoS Genet 3, e134). In addition, the CIN assay allows measuring and distinguishing between chromosome breaks and chromosome loss. The CIN assay has also proved useful to test CIN in the presence of cell cycle drugs and in cell cycle mutants. It should also be a very useful tool to analyze chromosomal instabilities in all kinds of physical, chemical or genetic conditions.

Because of the scale down, multiple samples can be run easily and allow repetition and statistical analysis of the data, as well as lowering considerably the cost of the assay. Due to its increased sensitivity, the CIN assay can be performed on single colonies (usually ranging from about 1 to 10 million of cells per colony). Importantly, the CIN assay allows starting from a unique cell. It is therefore a clonal assay which can detect early events in chromosome instability.

### EXAMPLE 3: Use of the CIN assay for studying the effects of respiratory deficiency on nuclear genome stability

### 3.1. Introduction

Eucaryotic cells generally rely largely on mitochondrial oxidative phosphorylation (OXPHOS) for their ATP- supplies. However, cells can proliferate without OXPHOS, using the glycolytic/fermentation pathway, with a much reduced ATP/glucose yield. This process is common in baker yeast grown on high glucose, or in cells grown in absence of oxygen. It is also the fate of cells harboring mutations disabling the respiratory-chain (either nuclear encoded genes, leading to "petites", or mutations/loss of mitochondrial DNA, termed p-/0). Fermentation has also been described in cancer cells, as early as in the 1920's, a feature known as the "Warburg effect". Once considered a side-effect of cancer growth conditions, this metabolic change from respiration to fermentation has raised a lot of interest. Growing evidences point to a genetically controlled switch, with an important role in tumorigenesis. Mitochondria dysfunction and decreased respiration rates are also associated with aging. Hence, a subset of eukaryotic cells function in the absence of OXPHOS, due to physiological conditions, mutations or controlled metabolic switches.

Oxidative stress is a major issue for most organisms. Mitochondria are both the main producer of oxidants and primary targets of the oxidative damage. Reactive oxygen species (ROS) are mainly produced through leakage of electrons from the OXPHOS chain, and the 'free-radical theory of aging' proposed that oxidative damage over time is responsible for cellular aging. ROS can damage a wide variety of targets (proteins, fatty acids, nucleic acids...) but low levels of ROS can also have beneficiary effects on cell survival and serve as second messengers. Production of highly reactive hydroxyl radical from hydrogen peroxide or superoxide radical can lead to DNA strand breaks. In wild type yeast cells grown under standard conditions, ROS has been shown to be an important cause of chromosome instabilities (CIN).

In a yeast genome-wide study, mitochondria-deficient cells came out as the main category of mutants hypersensitive to external oxidative stress (Thorpe et al. 2004 Proc Natl Acad Sci U S A 101, 6564-6569). However, the cause of that sensitivity is unknown. In HeLa cells, mitochondrial defects correlates with impaired DNA repair in the nucleus, an observation that might explain their sensitivity to DNA damage by hydrogen peroxide.

In summary, ROS are a threat in both wild type and in respiratory-defective (RD) cells, but the latter show an increased sensitivity to oxidative stress. A few elements suggest that this phenomenon may be due to impaired repair capacities, but the molecular details remain unknown.

What, if any, is the consequence of OXPHOS inactivation on the maintenance of nuclear genome integrity? Quantitatively, RD yeast cells display a small increase in mutation rate compared to wild type. Mutations affecting mitochondria OXPHOS are found in some cancer cells, sometimes associated with gross chromosomal modifications. A model linking mitochondria dysfunction and nuclear genome instability has therefore been put forward and discussed. However, quantitative data showing a strong impact of respiratory dysfunction on nuclear genome stability is missing in support of this model.

In the examples herebelow, the potential effects of respiratory deficiency on nuclear genome stability have been addressed in quantitative terms.

### 3.2. Respiratory-defective cells display elevated CIN

Mutations in many of the genes involved in mitochondrial functions lead to inactivation (directly or indirectly) of the respiratory electron transport chain (ETC). Those include nuclear mutations (*"petite"*) or mitochondrial ones (p- for partial and ρ0 for total loss of mtDNA). All have in common a crippled mitochondrial respiratory chain. Those respiratory-defective (RD) cells are characterized by their inability to grow on non-fermentable carbon source.

For this study, RD cells from three sources were analyzed: (a) ρ0 cells (b) a *mip1* mutant, defective for mitochondrial-specific DNA polymerase (Genga et al. 1986 J Biol Chem 261, 9328-9332) and (c) *pet* mutant, a natural occurring *petite* strain. None of these strains had visible mitochondrial DNA by DAPI stain. Strikingly, when tested using a CIN assay, all three types of RD cells showed a significant increase in nuclear genome instability, from 5 to 30 fold. The chromosome instabilities corresponded to both chromosome breaks and losses.

In previous studies, point mutations in RD cells had been measured and a mild increase had been observed (Rasmussen et al. 2003 Nucleic Acids Res 31, 3909-3917). Here, mutation frequencies were measured using haploid parental strains. It was shown that mutation frequencies in *URA3* gene reached the expected value (around 1 10⁻⁶ in wild type RC cells). Identical values were measured in ρ0 RD cells, with broad standard deviation, as expected for stochastic events. Hence, unlike chromosome instability, which is significantly higher in RD ρ0 cells, point mutations frequencies at the *URA3* locus are similar in RC and RD cells.

### 3.3. Sensitivity of RD cells to cell cycle drugs and oxidative stress

Genome instability as described above has been observed in various mutant contexts. Two main types are (a) cell cycle mutants, affecting "the cell cycle engine", and (b) "checkpoint" or surveillance mechanism mutants (Hartwell and Weinert 1989 Science 246, 629-634). Defects in the DNA replication machinery is a major contributor to this type of instability, as S phase is probably the most sensitive period challenging DNA integrity during cell proliferation. Although usually not essential for cell cycle progression, this class of genes is important to maintain the fidelity of cycle events, hence an increase of genomic instability observed in those mutants (recently reviewed in Aguilera and Gomez-Gonzalez 2008 Nat Rev Genet 9, 204-217).

Because of their function, checkpoint mutants are often sensitive to various cell cycle drugs (affecting S and/or M phase). If RD cells have high CIN because of a putative checkpoint defect, they are expected to be sensitive to drugs challenging the corresponding surveillance mechanism. The effect of cell cycle drugs, including S-phase inhibitor (HU), DNA alkylating (MMS) agents, DNA double-strand break formation (bleomycin) and microtubule poison (benomyl) was therefore tested on RD strains. As control strains, wild type (checkpoint proficient) and checkpoint mutants for DNA damage/DNA replication *mec1 sml1* and spindle checkpoint mutant *bub1* were used.

It was demonstrated that RD cells (ρ*0, mip1, mrps28*) were not particularly sensitive to any of the tested drugs. This suggests that RD cells have no major defects sensing, signaling or repairing these types of damages. A mild sensitivity was observed with the DNA damaging agent bleomycin. However, the same RD cells showed striking growth defects when plated on media inducing an oxidative stress (hydrogen peroxide, 4 and 6 mM). Oxidative stress blocked RD cells quickly, at unbudded or single budded stage. Thus most cells did not complete a single cell cycle (ρ*0, mip1, mrps28*), while WT and bub1 controls formed colonies in the same conditions. Surprisingly, respiratory competent *mec1 sml1* cells were as sensitive as RD cells to H₂O₂, a fact worth noting. These cells arrested mostly as budded cells in the first cell cycle. Hence, RD cells seem particularly sensitive to oxidative stress but not to other tested cell cycle drugs.

### 3.4. Internal oxidative damage is the likely source of instability in RD cells

RD cell's lethality is observed when external oxidative stress is applied. One could wonder whether internal ROS damage is responsible for the elevated CIN observed earlier in unperturbed RD cells. If so, one might expect to reverse this effect by a mere addition of an anti-oxidant to the medium. It is indeed the case.

Addition of the oxygen scavenger N-acetyl cysteine (NAC) accelerated the growth of RD cells in a dose dependent manner. More strikingly, NAC addition to the medium fully reversed high CIN of RD cells 30 fold, down to the control wild type level. Addition of NAC to WT control cells neither led to accelerated growth nor to chromosome stabilization.

Hence, RD cells grow faster and with far less genomic instability in the presence of an anti-oxidant. This suggests the presence of an ill-processed internal oxidative stress in mitochondria-defective cells, leading both to slow growth and to high genomic instability in standard conditions.

To further explore this idea, a major ROS detoxifying enzyme, *TSA1* (Chae et al. 1994 J Biol Chem 269, 27670-27678), was deleted both in respiratory-competent (RC) and respiratory-defective (RD) backgrounds. The mutant was then challenged them with low H₂O₂ doses (1 mM).

This concentration did not increase CIN, neither in RC *TSA1₊* cells nor in RD *TSA1₊* cells. *tsa1* RC cells showed high CIN (131 +/- 18 10⁻⁶, 20 fold above wild type RC control). Low dose (1 mM) of H2O2 increased CIN only a little further (204 +/- 27 10⁻⁶), indicating that these cells are still capable of dealing with this stress (either by detoxification and/or repair). RD cells showed a different behavior. Firstly, the basal level of CIN in *tsa1* RD was higher (456 +/- 98 10⁻⁶, 76 fold above wild-type CIN levels). Secondly, the mild H₂O₂ treatment further increased this instability to extreme proportions (3600 +/- 346 10⁻⁶, 600 fold above wild-type CIN level). Thus, when crippled for a main anti-oxidant defense, RD cells, unlike RC, prove very inefficient in maintaining genomic stability in the face of a low external oxidative stress.

### 3.5. Superoxide levels are low in RD ρ0 cells

Experiments above clearly point to oxidative damage as a source of instability for genomes of RD cells, leading even to lethality when external ROS are applied. Hydrogen peroxide levels (measured using scopoletin) are elevated in ρ0 cells as well as in various *pet* mutants (using AmplexRed detection).

To determine the level of superoxide anions in RD cells, oxidation of DHE was measured in the three RD strains mentioned above. A clear ten fold decrease of superoxide anions was seen in RD ρ0 compared to wild type. This result is consistent with the article by Rasmussen et al. (2003 Nucleic Acids Res 31, 3909-3917). Thus, RD cells undergo ROS-dependent CIN, despite low internal superoxide levels but rather elevated H₂O₂ levels.

### 3.6. Signs of chromosome breaks are visible in most cells of an RD population

To have a more global view of the damage in the populations, a cytological marker was used. The Rad52p protein is present and concentrates at sites of DNA damage (double-strand breaks). Using fluorescence microscopy, a Rad52-GFP fusion can be used to detect repair-foci *in vivo*. Thus yeast strains harboring an integrated *RAD52::GFP* fusion construct were analyzed by fluorescence microscopy of life cells (Lisby et al. 2001 Proc Natl Acad Sci U S A 98, 8276-8282).

In a wild-type RC population grown under unperturbed conditions, about 5% of the cells display such foci, mostly arising from chromosomal breaks during S-phase. Strikingly, in a respiratory-defective population harboring the Rad52::GFP fusion protein, clear foci are visible in every second cell of ρ0 RD (52%), a very high percentage both compared to wild type (10 fold increase) and to other known unstable mutants like *mec1* (22%), the latter being a central DNA damage checkpoint gene. Thus, DNA damage seems widely present in ρ0 RD cells cycling in unperturbed conditions.

### 3.7. Conclusion

The data show that the nuclear genome becomes highly destabilized in respiratory-deficient cells (chromosome instabilities rather than point mutations). The data strongly suggest that the source of this instability is due to oxidative stress in RD ρ0 cells.

## Claims

1. A yeast strain for measuring chromosome instability comprising a first and a second selectable marker located on the same arm of a chromosome, wherein said first and second selectable markers are negative selectable markers, **characterized in that**:
- said yeast strain is diploid or polyploid;
- said yeast strain further comprises a third selectable marker located on said chromosome; and
- said first, second and third selectable markers are only present on one of the homologous copies of said chromosome.

2. The yeast strain according to claim 1, wherein said yeast strain is diploid.

3. The yeast strain according to claim 1 or 2, wherein the first and second selectable markers are linked together.

4. The yeast strain according to any one of claims 1 to 3, wherein the first and second selectable markers are located in the most centromere-distal third of said arm, and wherein the third selectable marker is located in the most centromere-proximal third of the same or of the opposite arm.

5. The yeast strain according to any one of claims 1 to 4, wherein:
- the first and second selectable markers are located at a distance of at least 50 kb to the centromere; and
- the third selectable marker is located at a distance of less than 50 kb to the centromere.

6. The yeast strain according to any one of claims 1 to 5, wherein:
- said first selectable marker is *CAN1;*
- said second selectable marker is *URA3*; and/or
- said third selectable marker is *LEU2.*

7. The yeast strain according to any one of claims 1 to 6, wherein said chromosome is chromosome V.

8. A method for measuring chromosome instability comprising the steps of:
a) providing a yeast strain according to any one of claims 1 to 7;
b) plating cells of said yeast strain on a medium selecting cells in which said first and second selectable markers have been lost; and,
c) replicating cells growing at step (b) on a medium selecting cells in which said third selectable marker is either present or absent.

9. The method according to claim 8, wherein step (a) further comprises the step of growing said yeast strain on a non-selective medium.

10. The method according to claim 9, wherein said yeast strain is grown on said non-selective medium in the presence of a compound susceptible of playing a role in chromosome instability.

11. The method according to claim 9 or 10, wherein said yeast strain is **characterized in that** a gene susceptible of playing a role in chromosome instability is deleted, disrupted, inactivated or overexpressed in said yeast strain.

12. The method according to any one of claims 9 to 11, wherein step (a) further comprises the steps of isolating a colony and suspending cells of said colony in a solution.

13. The method according to any one of claims 8 to 12, wherein said third selectable marker is a positive selectable marker, and wherein the medium of step (c) selects cells in which said third selectable marker is present.

14. The method according to claim 13, wherein :
- the detection of a colony growing both on the medium of step (b) and on the medium of step (c) indicates that a chromosome break event has occurred in the cells of said colony; and/or
- the detection of a colony growing on the medium of step (b) but not on the medium of step (c) indicates that a chromosome loss event has occurred in the cells of said colony.

15. Use of the strain according to any one of claims 1 to 7 for measuring chromosome instability.
